# EUROPEAN PATENT SPECIFICATION

(11) **EP 0 613 510 B1**
(45) Date of publication and mention of the grant of the patent: **21.01.1998**
(21) Application number: 92922901.1
(22) Date of filing: 11.11.1992
(51) Int. Cl.: D21H 19/28, D21H 23/66, B05D 1/06

(54) **PROCESS FOR MAKING A STRUCTURE HAVING CONTROLLED WATER RESISTANCE**
VERFAHREN ZUR HERSTELLUNG EINER STRUKTUR MIT EINER KONTROLLIERTEN WASSERBESTAENDIGKEIT
PROCEDE DE FABRICATION D'UNE STRUCTURE PRESENTANT UNE RESISTANCE A L'EAU CONTROLEE

(30) Priority: 12.11.1991 GB 9123997
(43) Date of publication of application: 07.09.1994
(73) Proprietor: MONSANTO COMPANY, St. Louis, Missouri 63167 (US)
(72) Inventor: BRUNGER, Peter, Maurice 10 Aylsham Close, Stockton on Tees TS17 0UP (GB); KEMMISH, David, John, Thirsk YO7 1EP (GB)
(74) Representative: Nash, Brian Walter
(86) International application number: PCT/GB92/02078
(87) International publication number: WO 93/10308

(56) References cited:
- WO-A-91/13207
- WO-A-92/01733
- US-A- 3 657 044

## Description

THIS INVENTION relates to a process for making a structure comprising a core of a material having mechanical strength when dry but not when water-wet and a coating comprising biodegradable water-resistant polymer.

WO 91/13207 describes the production of such structures by applying a latex comprising a colloidal suspension in water of essentially non-crystalline particles of a polymer or copolymer to paper or board substrates by dipping or spraying. The process of the present invention involves the electrostatic application of dry fine particles rather than the application of a latex.

The process of the present invention is a process for making a structure comprising a core of a material having mechanical strength when dry but not when water-wet and a coating comprising water-resistant polymer by electrostatic application of dry fine particles of such polymer, in which process
(a) the polymer is a biodegradable microbiologically produced polyhydroxyalkanoate consisting of repeating units of formula O-R-CO where R is an aliphatic chain of 2 to 6 carbon atoms optionally carrying a C₁-C₄ branch on the carbon atom next to oxygen; and
(b) the dry fine particles of polymer are particles as laid down in microorganism cells or clusters of such particles, in each case after removal of cell debris.

The process of the present invention may also provide compound structures in which two or more structures are laminated together or in which core material or other material is laminated to the structure.

The core can be for example a foam or assemblage of particles or assemblage of fibres or any combination of these. Such fibres are preferably naturally occurring, rather than man made. The core thus could be bread-like or paper or papier-maché or felt or candyfloss-like. It may include one or more water-sensitive and/or biodegradable adhesives. There may be water-resistant material mixed in with water-disintegrable material, provided the proportion thereof is insufficient to decrease water-sensitivity below the level required for the intended use of the structure.

The coating can be, or can be convertible by heat treatment to, for example a smooth fused or sintered film covering the whole surface of the core, or a reticulated film following the contours of the core, or both. It can be limited, for example to the surfaces of the units (such as fibres) of which the core is composed, or to junctions between such units. The coating can be of thickness in the range 5 to 500 preferably 20 to 200 µm, for example. The coating of biodegradable polymer can be additional to a coating of water-sensitive material.

In one form of the invention the structure has one dimension substantially less than the other two, so that it has two sides: then it can carry the coating on both sides or only one side. The polymer coating can be the working surface of the structure. Alternatively the core material can be the working surface, for example, in a structure required to absorb water or grease from one side while the other side maintains its mechanical integrity during absorption. Such structures are subject to disintegration thereafter in a microbially active environment.

The structure is when dry preferably rigid or to a required extent flexible and resilient, e.g. to permit rolling-up.

Application of the polymer in fine particulate form may be followed by heating to fuse or sinter the polymer and, if appropriate, to increase its crystallinity.

The material of the core can be for example watersoluble, for example sugar, starch, polyvinyl alcohol or a water-sensitive ester or ether thereof or poly(meth) acrylic acid or a water-sensitive ester or amide thereof or a cationic vinyl or (meth)acrylic polymer. It may be slowly biodegradable, such as cellulose, eg wood pulp or paper, or non-delignified material such as wood shavings, sawdust, hay or straw. Very suitably it is paper or papier maché, especially recycled. For practical purposes it is thus to be regarded as at least water-disintegradable.

The polyhydroxyalkanoate molecular weight Mw is preferably over 100000, especially in the range 200000 to 1.5 million. In particular examples, referred to hereinafter as HB(HV), the units are 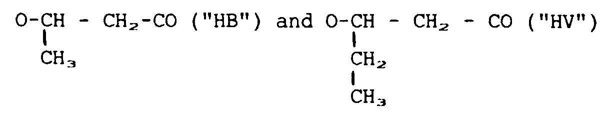 or both such units may be present. The relative proportion of such units is preferably such as will give a polymer that is crystallisable e.g. on holding at 20 to 100°C for 0.1 to 0.5h or possibly up to 4h. If copolymer is used it may contain for example at least 2, especially 3 to 25, mol percent of HV, balance HB. The proportion of HB and HV units may if desired be attained by blending for example polymer containing 0-5 mol% HV with polymer containing 5-30% HV.

These proportions disregard very small, possibly fractional, percentages of units containing more than 5 carbon atoms, which may be present.

The material of the coating may consist of such polymer or may be a blend with other polymer. The proportion of other polymer in the blend depends on how rapidly the coating is required to biodegrade, and on the extent to which the other polymer is itself biodegradable or biodisintegrable. Suitable blends are described in our EP-A-0052460 and PCT application 91/01733 claiming priority from GB application 9016345.2 filed 25 July 1990.

In addition to polymer(s) there may be present any of the usual polymer processing additives, for example one or more plasticisers, particulate fillers, reinforcing fibres, pigments and nucleating agents, subject to suitability for the method used for making the structure.

The core material may be shaped wet and then dried, or shaped dry with a fine-particulate fusible adhesive, then heat-set. Application of polymer is suitably to core material having a water content low enough for it to keep its shape during application, that is, in a "green" condition or more dry. It may be substantially dry, for example in equilibrium with ambient air at up to 90% relative humidity. If it contains an adhesive, the adhesive may be or include a biodegradable material, possibly a microbiologically produced polymer.

The polyhydroxyalkanoate is recovered by one of the following processes:
(1) the procedure described in EP-A-46335 involving heating an aqueous suspension of micro-organism cells containing polymer granules under pressure to above 100°C, particularly to above 150°C, and then releasing the pressure. This process causes the cell walls to rupture enabling the polymer granules to be separated from the cell debris by conventional methods;
(2) digesting dried cells, obtained for example by spraying an aqueous cell suspension with hypochlorite, for example as described in J.Gen. Microbiol. 19 (1958) 198-209.
By such processes polymer can be separated from the cell residue as granules as laid down in the cells or as clusters of such granules.
(3) digestion of the non-HB(HV) polymer material in the micro-organism cells, for example with a proteolytic enzyme composition. In this way the non-HB(HV) polymer cell material can be solubilised, leaving the HB(HV) in particulate form. In order to assist separation of the polymer from the aqueous medium, the aqueous suspension is preferably heated, particularly to above 100°C, prior to the enzyme digestion;

The microorganism producing the polymer can be any one of those capable of accumulating it, for example of the genera Alcaligenes, Athiorhodium, Azotobacter, Bacillus, Nocardia, Pseudomonas, Rhizobium and Spirillium, or others such as Escherichia or, alternatively a eukariote into which the necessary genetic material has been introduced. Alcaligenes, for example A.eutrophus or possibly A.latus is conveniently used.

The polymer used is preferably in particles of average size in the range 0.2 to 500, especially 0.5 to 200 µm. It is particularly advantageous that the polymer particles harvested from the micro-organism can be readily available within these size ranges. These ranges include particles as laid down in micro-organism cells and also clusters of such particles such as result from the separation technique and/or from spray drying a latex or solution. If clusters are used, their particle size is suitably in the range 5-100, especially 10 to 80 µm.

Electrostatic coating is employed by contacting the core with electrostatically charged polymer particles. This may be by immersion in a fluidised bed of such particles. If the core is to be coated on one side only, two cores may be sandwiched together before immersion, if their shape permits this. The core may be wet with water to facilitate earthing. Alternatively a metal ground plate may be applied to the side or parts of the core that are not to be coated. If the core has 3-dimensional shape the ground plate may be metal foil formed to its shape. However, coating can be successfully carried out without such earthing.

After application of polymer, the coated core may be stored or shipped. It is subjected preferably to one or both of the following heat treatments:
drying, if the core was wet;
setting, typically at 160 to 200°C for 0.1h;
lamination, if a multi-layer structure is required.
(The polymer coating after setting can be tacky, or become tacky in next following treatment, sufficiently for lamination without further adhesive, but further adhesive can be used if desired, preferably biodegradable or discontinuously applied);
crystallisation, typically at 40 to 75°C for 0.1 to 1h, possibly in contact with a solid surface such as a calender. Such surface may be for example mirror-polished, textured, profiled or embossed with a message, or may merely act as a heat transfer medium.

The invention provides a process and apparatus in which these operations are performed successively on a continuous web of core material, or in combination with an initial step of shaping or part-shaping, and drying if such initial step is of wet material.

The invention provides particular examples of the structure, namely
boards, sheets and dishes coated on one or both sides; vessels and pipes;
blanks to be heat set or crystallised or both;
blanks to be slit or chopped;
stripform or particulate litter or absorbent packing;
packaging, especially compartmented trays for water- or grease- containing foods;
personal hygiene products such as diapers, sanitary napkins, incontinence sheets or surgical swabs;
ostomy bags;
bed pans, urine bottles; or
any of these carrying a layer of core or polymer material on one or both sides.

Such structures preferably are disintegrable rapidly enough to be handled by domestic or hospital sewerage or wet waste disposal machines and composting systems and plants. They are capable of many duties now performed by foamed polystyrene, but in a more environmentally acceptable way.

### EXAMPLE 1 (Design)

For an experimental run the core is a pressed papier maché dish and the coating material is a polymer consisting of HB and HV units in the mol ratio 94 to 6 and having a molecular weight of over 200000, preferably over 350000. It was produced by spray drying an aqueous suspension resulting from the removal of cell debris from micro organism cells and had an average particle diameter of about 50 µm.

The powder is charged to an Electrostatic Fluidised Bed Coater (Electrostatic Technology Inc, Branford, Connecticut USA) and applied to outer side of the dish at coating weights corresponding (after the heat treatments to be described) to about 100 and about 25 µm thickness. The dish is removed from the Coater to an air oven at 180°C and kept at 180°C for a few minutes until the surface particles have fused together into a coherent film. The coated dish is then transferred to an air oven at 60°C and kept there for 4 min until the polymer coating has crystallised.

From the results of dummy experiments preceding the above run it is expected that the dish will hold water for 5-10 min without collapsing and can then be disintegrated in sewerage, composting and other microbially active systems.

This was in fact confirmed. An egg carton coated on the outside was found to float on water for at least 10h. The corresponding uncoated control sank in about 10 min.

### EXAMPLE 2

Coating runs were carried out using a variety of core materials and microbiologically produced polymer of molecular weight Mw 643000 consisting of HB and HV units in the mol ratio 93 to 7 in the form of particles passing a 35 ASTM sieve but 98.6% w/w greater than 300 mesh. The polymer was applied using a Model C30 mini coater from the range of coaters mentioned in Example 1, operated in these conditions:

| | |
|---|---|
| Fluidised bed air velocity | 5.66 m³ min⁻¹ |
| Kilovolt setting | 80 |
| Immersion time | 4, 6 or 8 sec |

to weighed core specimens backed by a metal ground plate. The coated specimens were weighed, then heated at 180°C for 2 min to fuse the coating and at 60°C for 5 min to permit crystallisation, then tested for water resistance by observing whether one drop of water on the coating for one minute soaked in or not. Results for typical core materials are shown in Table 1.

**Table 1**

| Core Material | Wt% polyester applied | Water Resistance | Surface Quality |
|---|---|---|---|
| Kraft Paper | 87.5 | Yes | moderately rough |
| Kraft Paper | 55.6 | Yes | moderately rough |
| Egg board (papier maché) | 44.4 | Yes | rough |
| Egg board (papier maché) | 17.0 | Yes | rough |

The coated specimens are ready for use as for example disposable water-containing vessels. If smoothness is required the coating may be calendered or otherwise smoothed.

### EXAMPLE 3

Example 2 was repeated using a polymer consisting of HB and HV units in the mol ratio 81 to 19 and having Mw 678000. Owing to slow crystallisation the coated specimens were held at ambient temperature for 1h before testing. Results for typical core materials are shown in Table 2.

**Table 2**

| Core Material | Wt% polyester applied | Water Resistance | Surface Quality |
|---|---|---|---|
| Kraft Paper | 244.4 | Yes | moderately rough |
| Egg board (papier maché) | 95.0 | Yes | rough |
| Fibreglass Cloth | 20.8 | Not Tested | Smooth |

### EXAMPLE 4

The procedure of Example 2 was repeated using polymer consisting of HB and HV units in the mol ratio 83 to 17. For a single core material (papier maché egg carton) the following variables were explored:
(a) effect of aluminium foil earth pressed to egg carton contour;
(b) effect of immersion time 4, 6 or 8 sec;
(c) effect of kilovolt setting.

For each coating operation the air flow rate was 5.1 m³ min⁻¹. Crystallisation was apparently complete within 10 min at 60°C.

A representative selection of the coating weights obtained is shown in Table 3.

**Table 3**

| Aluminium Foil Earth | Immersion time, sec | Kilovolt setting | Coating % w/w |
|---|---|---|---|
| - | 4 | 60 | 45.7 |
| + | 4 | 60 | 38.2 |
| - | 4 | 50 | 30.8 |
| - | 6 | 50 | 42.8 |
| - | 8 | 50 | 52.3 |
| - | 4 | 40 | 8.0 |
| - | 4 | 50 | 30.8 |
| - | 4 | 60 | 29.7 |
| - | 4 | 70 | 40.1 |
| - | 4 | 80 | 62.3 |

By visual examination the optimal coating weight was 30-50% : such specimens could be flexed without cracking the coating, had a uniform appearance and resisted penetration by a water drop for at least 1 min. The lower coating weight obtained using the aluminium foil earth is attributed to preferential attraction of smaller particles.

### EXAMPLE 5 : Triboelectric coating

Pressed papier maché sheets 120 x 70 mm x 1.25 mm were coated on one side with polymer consisting of HB and HV units in the mol ratio 84.4 to 15.6 having Mw 646000, melt flow index 10 at 190°C using a Davenport MFI Grader, and peak melting point 145°C as calculated using a Perkin Elmer DSC4 instrument.

The polymer, in the form of powder produced by spray drying an aqueous suspension resulting from the removal of cell debris from A.eutrophus cells, was sieved to a particle size range 60-200 µm. It was placed in a small tank, fluidised by blowing air in from below, sucked from the fluidised bed by a Venturi effect and blown along a plastic tube into the inlet of a Ransburg Gema triboelectric gun. Within the gun the particles acquired electrostatic charge by frictional contact with PTFE surfaces. The emerging powder was directed onto the papier maché sheets hanging in an earthed frame. A range of spray times in the range 5 to 30 sec was used, to provide a range of coating weights. The sprayed sheets were cured in a fan-assisted oven at 200°C for 20 min, then held at 60°C for 4 min to permit crystallisation. The water-proofing effectiveness of the coating was tested by placing a drop of Lugol's reagent (potassium iodide + iodine in aqeous ethanol) on it and noting the time taken for absorption to occur. Typical coating weights were in the range 45 to 200 g m⁻² and absorption times 30 to 125 min.

## Claims

1. A process for making a structure comprising a core of a material having mechanical strength when dry but not when water-wet and a coating comprising water-resistant polymer by electrostatic application of dry fine particles of such polymer, in which process
(a) the polymer is a biodegradable microbiologically produced polyhydroxyalkanoate consisting of repeating units of formula O-R-CO where R is an aliphatic chain of 2 to 6 carbon atoms optionally carrying a C₁-C₄ branch on the carbon atom next to oxygen; and
(b) the dry fine particles of polymer are particles as laid down in microorganism cells or clusters of such particles, in each case after removal of cell debris.

2. A process according to claim 1 in which the core is an assemblage of fibres.

3. A process according to claim 2 in which the core is of paper-mach.

4. A process according to any one of the preceding claims in which the coating is 20 to 200 µm thick.

5. A process according to any one of the preceding claims in which the polyhydroxyalkanoate contains 3 to 25 mol percent of hydroxyvalerate residues, the balance being hydroxybutyrate residues.

6. A process according to any one of the preceding claims in which the core has initially a 3-dimensional shape and in which there is applied to it, before coating, a metal foil ground plate formed to its shape.

## Patentansprüche

1. Verfahren zur Herstellung einer Struktur, umfassend einen Kern aus einem Material mit mechanischer Festigkeit im trockenen, jedoch nicht im wasserfeuchten Zustand und eine ein wasserbeständiges Polymer umfassende Beschichtung durch elektrostatisches Aufbringen trockener, feiner Teilchen eines solchen Polymeren, wobei bei diesem Verfahren
(a) das Polymer ein bioabbaubares, mikrobiologisch erzeugtes Polyhydroxyalkanoat ist, das aus wiederkehrenden Einheiten der Formel O-R-CO besteht, worin R eine aliphatische Kette mit 2 bis 6 Kohlenstoffatomen ist, die wahlweise eine C₁-C₄-Verzweigung an dem Kohlenstoffatom, das dem Sauerstoff am nächsten ist, trägt; und
(b) die trockenen, feinen Teilchen aus dem Polymeren Teilchen sind, wie sie in Mikroorganismuszellen festgelegt sind, oder Cluster solcher Teilchen sind, jeweils nach Entfernung der Zelltrümmer.

2. Verfahren nach Anspruch 1, wobei der Kern ein Faseraufbau ist.

3. Verfahren nach Anspruch 2, wobei der Kern aus Pappmaché besteht.

4. Verfahren nach mindestens einem der vorangehenden Ansprüche, wobei die Beschichtung 20 bis 200 µm dick ist.

5. Verfahren nach mindestens einem der vorangehenden Ansprüche, wobei das Polyhydroxyalkanoat 3 bis 25 Mol% Hydroxyvaleratreste enthält, wobei der Rest Hydroxybutyratreste sind.

6. Verfahren nach mindestens einem der vorangehenden Ansprüche, wobei der Kern anfänglich eine dreidimensionale Form aufweist, und wobei an diesem vor der Beschichtung eine auf dessen Form gebildete Metallfolien-Erderplatte angebracht wird.

## Revendications

1. Procédé de fabrication d'une structure qui comporte une âme en un matériau qui présente une certaine résistance mécanique lorsqu'il est sec, mais n'en présente plus lorsqu'il est mouillé par de l'eau, et un revêtement comportant un polymère résistant à l'eau, par dépôt électrostatique de fines particules sèches d'un tel polymère, dans lequel procédé
a) le polymère est un poly(hydroxyalcanoate) biodégradable, produit par des micro-organismes, constitué de motifs répétés de formule -O-R-CO- où R représente une chaîne aliphatique de 2 à 6 atomes de carbone parmi lesquels l'atome de carbone voisin de l'atome d'oxygène peut porter, le cas échéant, une ramification en C₁₋₄, et
b) les fines particules sèches de polymère sont des particules qui se trouvent dans l'état où elles se sont formées dans des cellules de micro-organismes, ou des amas de telles particules, mais dans l'un ou l'autre cas, après élimination des débris cellulaires.

2. Procédé conforme à la revendication 1, dans lequel l'âme est constituée d'un assemblage de fibres.

3. Procédé conforme à la revendication 1, dans lequel l'âme est en papier mâché.

4. Procédé conforme à l'une des revendications précédentes, dans lequel le revêtement est épais de 20 à 200 µm.

5. Procédé conforme à l'une des revendications précédentes, dans lequel le poly(hydroxyalcanoate) comporte de 3 à 25 % en moles de motifs de type hydroxyvalérate, le reste étant constitué de motifs de type hydroxybutyrate.

6. Procédé conforme à l'une des revendications précédentes, dans lequel l'âme possède initialement une forme tridimensionnelle et l'on applique contre l'âme, avant d'effectuer l'opération de revêtement, une plaque servant de terre, qui est une mince feuille de métal épousant la forme de l'âme.
